# EUROPEAN PATENT SPECIFICATION

(11) **EP 3 325 963 B1**
(45) Date of publication and mention of the grant of the patent: **02.09.2020**
(21) Application number: 16828629.2
(22) Date of filing: 22.07.2016
(51) Int. Cl.: G01N 33/48, A61B 5/00, A61B 5/024, A61B 5/11, A61K 39/12, G16H 50/50

(54) **ENHANCING BLOOD CELL ESTIMATION**
VERBESSERTE BESTIMMUNG DER BLUTZELLEN
AMÉLIORATION DE L'ESTIMATION DE GLOBULES

(30) Priority: 23.07.2015 US 201562195837 P; 21.07.2016 US 201615216603
(43) Date of publication of application: 30.05.2018
(73) Proprietor: Transfusion Health LLC, South San Francisco, CA (US)
(72) Inventor: COTARI, Jesse, San Francisco, California 94114 (US); JACOBSON, Stuart Alexander, San Francisco, California 94110 (US); WEBB, Timothy, Concord, California 94520 (US); REBO, Justin, Crockett, California 94525 (US)
(74) Representative: Greaves Brewster LLP
(86) International application number: PCT/US2016/043641
(87) International publication number: WO 2017/015589

(56) References cited:
- WO-A1-2014/086803
- WO-A2-2012/042437
- WO-A2-2015/054701
- US-A1- 2004 122 706
- US-A1- 2011 285 529
- US-A1- 2014 066 884
- US-A1- 2015 184 222
- US-B1- 6 416 471

## Description

### TECHNICAL FIELD

The present technology pertains to immunotherapy, and more specifically pertains to enhancing the accuracy of a blood panel to account for activity-related changes to blood cell mobilization.

### BACKGROUND

Blood cells, such as stem cells, blood progenitors, red blood cells and all major types of white blood cells, are mobilized by activity. Blood analysis is a routine practice for monitoring general health and for screening for certain medical conditions and disorders. However, there is currently no solution for accounting for high variance in cell count, changes in concentration of certain blood cell types, changes in surface appearance of blood cells, etc. that are due to pre-test activity. In fact, even moderate activity, such as walking a flight of stairs, before a blood draw can alter the results of a complete blood count. Therefore, there is a need in the art for enhancing the accuracy of a blood panel by accounting for changes to blood cell mobilization due to activity and by accounting for individual variance in blood cell mobilization.

Document US 2014/066884 A1 discloses a pancreatic cell based model of blood glucose level including an individual's activity.

### SUMMARY

The scope of the present invention is solely defined by the appended claims. Additional features and advantages of the disclosure will be set forth in the description which follows, and in part will be obvious from the description, or can be learned by practice of the herein disclosed principles. The features and advantages of the disclosure can be realized and obtained by means of the instruments and combinations particularly pointed out in the appended claims. These and other features of the disclosure will become more fully apparent from the following description and appended claims, or can be learned by the practice of the principles set forth herein.

Disclosed are systems, and non-transitory computer-readable storage media for enhancing blood cell estimation. According to the invention, the present technology involves constructing a clinically-derived blood cell mobilization prediction model. The clinically-derived blood cell mobilization prediction model can involve correlating blood sample data from participants in a statistical sample of participants over a period of time with the participants' personal data relating to blood cell mobilization response, genetic data relating to blood cell mobilization response, and activity data collected from the participants.

According to the invention, personalized blood cell mobilization prediction models are constructed using the clinically-derived blood cell mobilization prediction model and a set of collected individual data. The collected individual data can include personal data relating to blood cell mobilization response, genetic data relating to blood cell mobilization response, and pre-test activity data for the individual. Additionally, the personalized blood cell mobilization prediction models can be further personalized by analyzing a set of historical blood sample data for the individual and historical activity data for the individual and determining whether to validate or invalidate one or more correlations in the clinically-derived blood cell mobilization prediction model based on one or more actual changes in mobilization rates for one or more blood cell and one or more actual changes to blood cell surface features observed in the historical blood sample data.

Also, according to the invention, blood cell mobilization prediction models are applied to enhancing the understanding of a blood test by predicting contemporary effects of pre-test activity data and creating an enhanced blood panel by excluding the contemporary effect of the contemporary activity from the contemporary blood sample data.

According to a non-claimed example, the blood cell mobilization prediction models can be applied to optimizing a vaccine, administration of a medicine, and other medical treatment. In some cases, a collection of antigens present in a vaccine is identified and an activity regimen for maximizing a concentration of a collection of immune cells which interact with the collection of antigens present in the vaccine can be prescribed.

The present technology can also involve enhancing the reliability of activity data by accounting for inconsistencies, gaps in data, etc. that is collected from telemetric devices. In some cases, when an inconsistency is detected in activity data, additional activity data can be collected from an additional telemetric sensor and can be substituted for the inconsistent data. Likewise, the technology can involve substituting missing data with average activity data from other days, an extrapolation of telemetry preceding and following a gap in data, etc.

### BRIEF DESCRIPTION OF THE DRAWINGS

In order to describe the manner in which the above-recited and other advantages and features of the disclosure can be obtained, a more particular description of the principles briefly described above will be rendered by reference to specific embodiments thereof which are illustrated in the appended drawings. Understanding that these drawings depict only exemplary embodiments of the disclosure and are not therefore to be considered to be limiting of its scope, the principles herein are described and explained with additional specificity and detail through the use of the accompanying drawings in which:
FIG. 1 illustrates a collection of data showing changes in white blood cell concentration in an individual over time after periods of exercise;
FIG. 2 illustrates a method of constructing a clinically-derived blood cell mobilization prediction model;
FIG. 3 illustrates a method of creating a personalized blood cell mobilization prediction model;
FIG. 4 illustrates a method of creating an enhanced blood panel;
FIG. 5 illustrates a system for creating and refining mobilization prediction modules using data gathered from a distributed plurality of user devices;
FIG. 6A illustrates a method of optimizing a vaccine;
FIG. 6B illustrates the results of an experiment comparing Hepatitis B Antigen positive cells (HBsAg+) per one million Peripheral Blood Mononuclear Cells (PBMC);
FIG. 6C illustrates the results of an experiment comparing a percentage of Hepatitis B Antigen positive cells (HBsAg+);
FIG. 6D illustrates a fold increase in the results of an experiment comparing a percentage of Hepatitis B Antigen positive cells (HBsAg+);
FIG. 7 illustrates a method of automatically compensating for inconsistencies and gaps in sensor data, ; and
FIG. 8A and FIG. 8B illustrate exemplary possible system embodiments.

### DESCRIPTION

Various embodiments of the disclosure are discussed in detail below. While specific implementations are discussed, it should be understood that this is done for illustration purposes only. A person skilled in the relevant art will recognize that other components and configurations may be used.

As explained above, blood cells, such as stem cells, blood progenitors, red blood cells, white blood cells, can be effectively mobilized through human activity. Mobilization of mature immune cells and immune progenitor cells can be correlated to baseline health and exercise metrics such as heart rate, exertion, etc.. Changes in circulation, changes in immune cell proteins and surface structure, etc. can be achieved by using brief, moderate to high intensity aerobic exercise. In some cases, even short exercise times (e.g. less than one minute) result in some mobilization. Also, in some cases, longer exercise can cause the concentration of white blood cells and stem/progenitors to decline more rapidly following an initial increase in concentration. Also, circulating immune system changes occur from almost any activity including light walking or even standing.

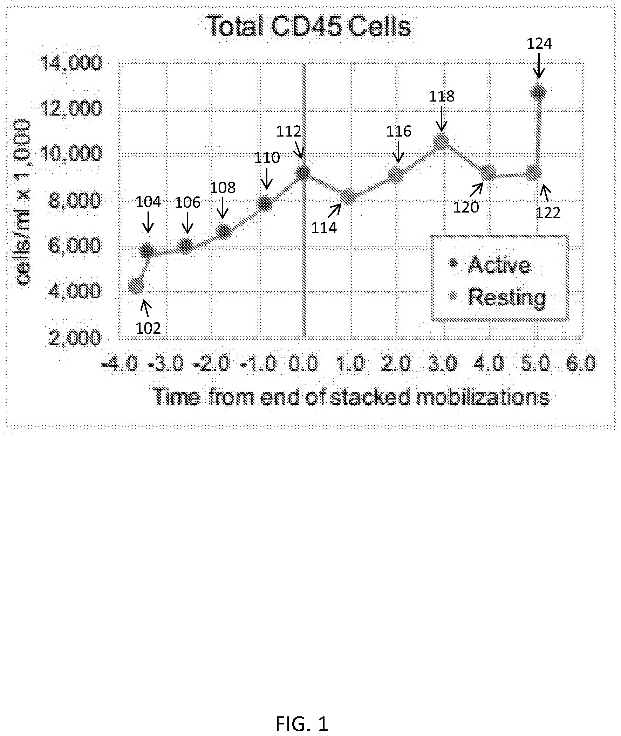

FIG. 1 illustrates a collection of data showing changes in white blood cell concentration in an individual over time after periods of exercise. The collection of data was derived using a regimen of supervised exercise and metered blood draws and post experiment analysis. As shown in FIG. 1, white blood cell concentration starts off on the lower end of normal and ends outside of the normal human white blood cell range. Data point 102 represents an initial resting data point before exercise commencement. Data points 104, 106, 108, 110, 112 represent active data points obtained from the individual after nine minutes of running. Data points 114, 116, 118, 120, 122 show further concentration of white blood cells in the individual during a further period of resting while the final active data point 124 was obtained after ninety seconds of running and shows a spike in white blood cell concentration.

FIG. 1 demonstrates how an individual with a white blood cell reading on the lower side of normal can induce an abnormal white blood cell reading using moderate activity. Such an observation also suggests that those individual's on the high end of normal may occasionally end up significantly outside of the "healthy range" during routine blood tests.

Failure to account for the effect of activity on circulating blood cell concentrations reduces the diagnostic and prognostic accuracy of standard blood tests and reduces the precision in predicting the effect of exercise on the immune system (using a prediction engine) immediately and over time in an individual. Conversely, knowledge of the effects of activity on blood cell mobilization can be used to enhance the accuracy of a blood test, can also be utilized to mobilize specific cells known to combat specific illnesses and disorders, and can be used to increase the effectiveness of a treatment (e.g. a vaccine, chemotherapy) by priming an individual's blood and recommending post-treatment activity to further enhance the treatment. Accordingly, some embodiments of the present technology involves using a clinically-derived blood cell mobilization prediction model and/ or a personalized blood cell mobilization prediction model along with activity data collected from telemetry to enhance the reliability of blood test and to mobilize specific blood cells.

A personalized blood cell mobilization prediction model can be constructed using a clinically-derived blood cell mobilization prediction model and collected user data. Some embodiments of the present technology involve creating the clinically-derived blood cell mobilization prediction model by observing mobilization following exercise and quantifying the effects using clinical analysis (e.g. cytometric assays) for a statistical sample of participants. General predictions of blood cell mobilization for a given activity using population averages can also combined with personal data, such as height, weight, gender, and age. The prediction of a user's mobilization can be further optimized by collecting data points of actual mobilization, to account for inter-individual variation in the effects of various modulations. To achieve more personalized mobilization models, some embodiments of the present technology involve obtaining and analyzing personal genetic information and blood analysis data from one or more blood draws and using the analyzed data to optimize predictions, quantify results, etc. An example of relevant genetic information can be mutations in functional blood receptors, such as growth, survival or homing proteins. An example of relevant blood analysis data can be a change in rate of the turnover of T cells and/ or natural killer (NK) cells observed from actual post-activity blood draws can provide further data points.

After collecting data for a statistical population, an immune-response prediction model can be created in a clinical setting and can be used to provide others in the same or similar populations with a prediction about how the same or similar activity will affect their own immune-response.

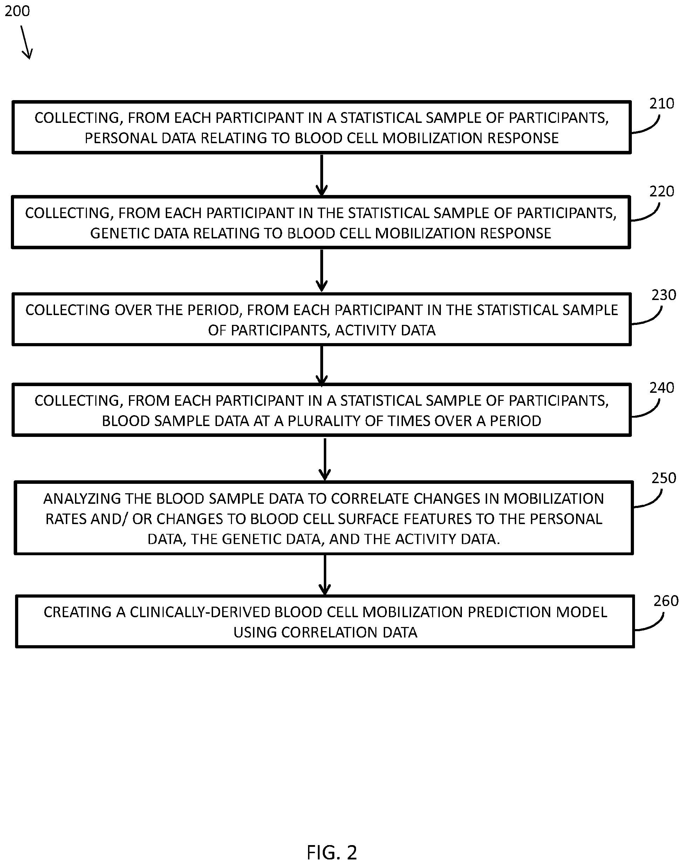

FIG. 2 illustrates a method 200 of constructing a clinically-derived blood cell mobilization prediction model. The method 200 involves collecting, from each participant in a statistical sample of participants, personal data relating to blood cell mobilization response 210. For example, personal data shown to relate to mobilization of blood cells includes personal factors such as height, weight, gender, ethnicity, age, etc. Additionally, the method 200 can involve collecting, from each participant in the statistical sample of participants, genetic data relating to blood cell mobilization response 220. For example, collected blood samples can be sequenced and analyzed by the same entity that constructs the clinically-derived blood cell mobilization prediction model; alternatively, participants' personal genetic data can be obtained from one or more commercial source of genetic sequencing and analysis.

The method 200 further involves collecting over the period, from each participant in the statistical sample of participants, activity data 230. In some cases, activity data can be self-reported and/ or automatically received from one or more telemetric devices associated with a participant, as explained in greater detail below.

Each of the personal data, genetic data, and activity data can include variables that are shown to correlate to changes in general mobilization of blood cells, mobilization of specific blood cells, surface structure of blood cells, etc. Therefore, a quantified degree in which these changes occur in the statistical sample of participants can be used to predict the likelihood that the variable will cause a similar change in the blood of other individuals. Therefore, the method 200 further involves collecting, from each participant in a statistical sample of participants, blood sample data at a plurality of times over a period 240 and analyzing the blood sample data to correlate one or more changes in mobilization rates for one or more blood cell and one or more changes to blood cell surface features to one or more factor selected from the personal data, the genetic data, and the activity data 250. Finally, the method 200 can involve creating the clinically-derived blood cell mobilization prediction model using the correlation data 260.

As explained above, the present technology also involves using the clinically-derived blood cell mobilization prediction model along with individual user data, an individual's activity data, and collected blood sample data to create a personalized blood cell mobilization prediction model. In some embodiments, the collection of data used to personalize a blood cell mobilization prediction model is gathered using telemetric devices worn or otherwise associated with an individual. Systems, methods, and non-transitory computer-readable storage media for creating, personalizing, and refining immune-response prediction models using telemetry and based on demographic user data, activity data, blood sample data, personal genetic information are described in further detail in co-pending U.S. Application Serial No. 15/215,465, filed on July 20, 2016, entitled "PREDICTING IMMUNE RESPONSE."

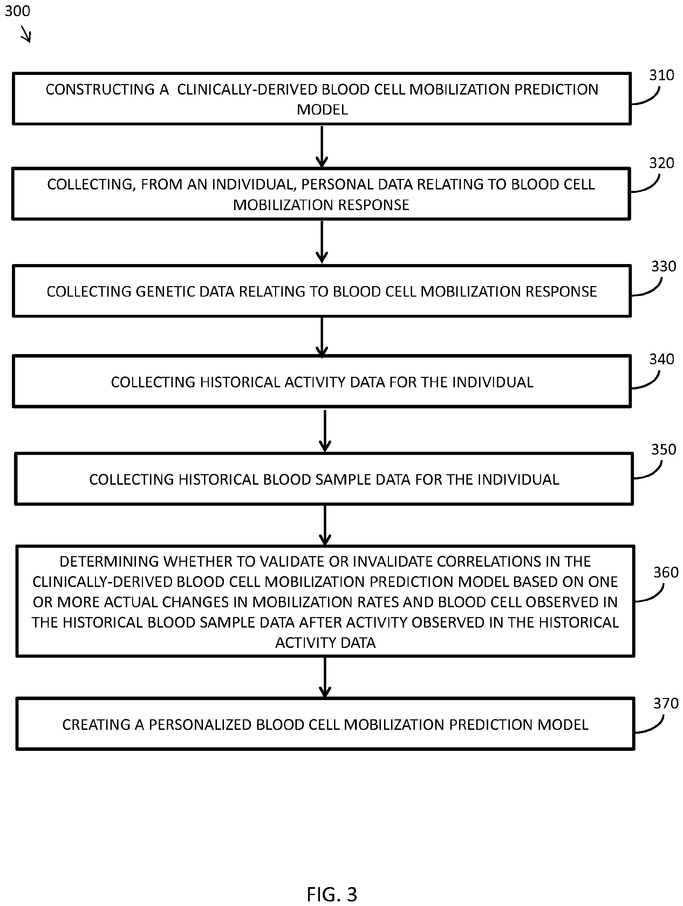

FIG. 3 illustrates a method 300 of creating a personalized blood cell mobilization prediction model. The method 300 involves constructing a clinically-derived blood cell mobilization prediction model 310 (e.g. according to the method 200). Next, the method 300 involves collecting, from an individual, personal data relating to blood cell mobilization response 320 and collecting the individual's genetic data relating to blood cell mobilization response 330. In order to analyze predicted correlations and validate or invalidate the correlations, the method 300 further involves collecting historical activity data for the individual 340 and collecting historical blood sample data for the individual 350.

Next, the method 300 involves determining whether to validate or invalidate correlations in the clinically-derived blood cell mobilization prediction model based on one or more actual changes in mobilization rates and blood cell observed in the historical blood sample data after activity observed in the historical activity data 360. Finally, using the correlation data and the validity or invalidity of the same, as observed in the actual changes in mobilization rates after activity, the method 300 involves creating a personalized blood cell mobilization prediction model 370.

A personalized blood cell mobilization prediction model has numerous applications that can benefit the health of an individual and that can be used by other institutions to monitor the well-being of an individual or group of individuals. In some cases, through the use of a smart device and telemetric sensors, an individual can observe change to their immune system and can determine the amount of exertion necessary to produce a desired effect. For example, an individual can determine an amount of exercise required to produce an effect on the circulating immune system in the immediate term and in the medium term (based on changes in circulating cells) and in the long term by shifting baselines and by influencing CD34 progenitors. Additionally, a health care provider can use a patient's personalized blood cell mobilization prediction model to make decisions about how to treat a patient. As explained above, knowledge of the effects of activity on mobilization can also be used to enhance the accuracy of a blood test.

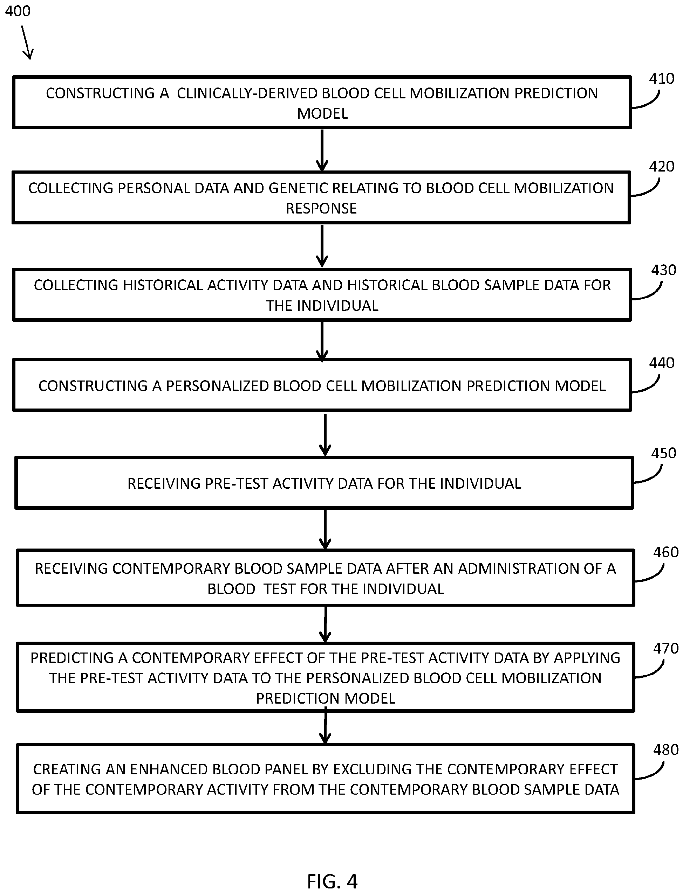

FIG. 4 illustrates a method 400 of creating an enhanced blood panel. First, the method 400 involves constructing a clinically-derived blood cell mobilization prediction model 410, collecting personal data and genetic relating to blood cell mobilization response 420, collecting historical activity data and historical blood sample data for the individual 430, and constructing a personalized blood cell mobilization prediction model 440.

Next, the method 400 involves receiving pre-test activity data for the individual 450. For example, a health care provider can "prescribe" a heart rate monitor, or other telemetric sensor device, to be worn by an individual in the days leading up to a blood draw. As explained in greater detail below, the health care provider can also prescribe a suggested pre-blood draw activity regimen.

Using the pre-test activity data and the personalized blood cell mobilization prediction model, a further prediction can be made about how the activity affected the blood cell counts, surface conditions, etc. of blood taken for a blood test (i.e. contemporary blood sample). Accordingly, the method 400 also involves receiving contemporary blood sample data after an administration of a blood test for the individual 460, predicting a contemporary effect of the pre-test activity data by applying the pre-test activity data to the personalized blood cell mobilization prediction model 470, and creating an enhanced blood panel by excluding the contemporary effect of the contemporary activity from the contemporary blood sample data 480.

Additionally, in some cases, through the use of telemetry before, during, and after a blood draw, a prediction that an increase in mobilized cells is due to tracked activity and the predicted effect on the results of the blood panel can be accounted for at time of blood draw.

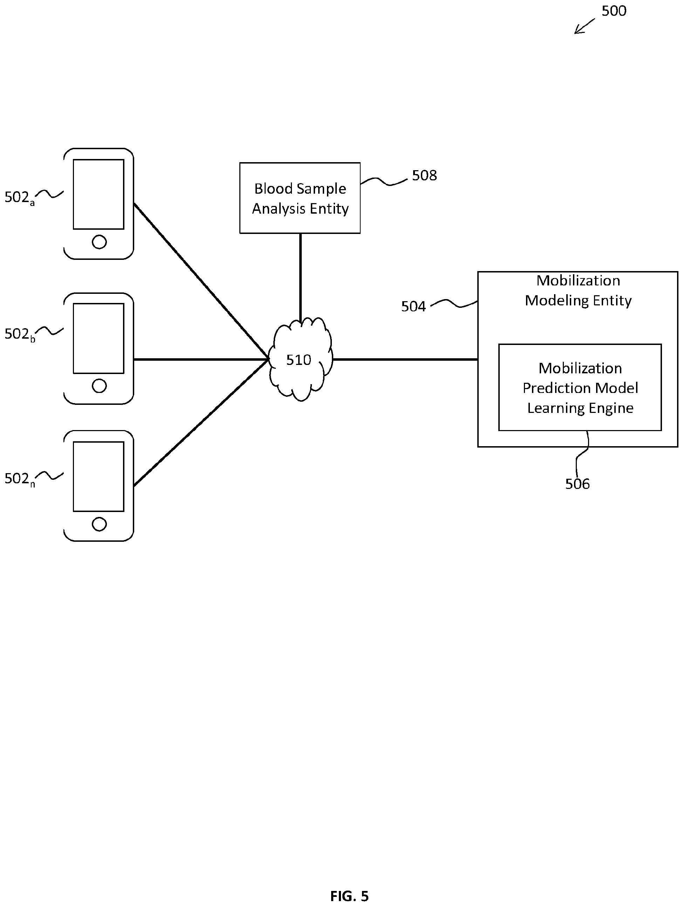

FIG. 5 illustrates a system 500 for creating and refining mobilization prediction modules using data gathered from a distributed plurality of user devices 502ₐ, 502_{b}, ..., 502ₙ. A user can opt-in to sharing personal information, activity data, and blood sample data and the shared data can be used to identify new insights and refine clinically created immune response models.

The system 500 includes a mobilization modeling entity 504 that uses clinical observations for a statistical population of participants to initially create a clinically-derived blood cell mobilization prediction model and to further refine the prediction model based on user feedback. The mobilization modeling entity 504 can distribute the clinically-derived blood cell mobilization prediction model to the plurality of user devices 502ₐ, 502_{b}, ..., 502ₙ that are connected to the immune-response modeling entity 504 via one or more network 510. Each of the plurality of user devices 502ₐ, 502_{b}, ..., 502ₙ can gather user information, activity data, and blood sample data from one or more blood sample analysis entity, personal genetic information, etc. Also, each of the plurality of user devices 502ₐ, 502_{b}, ..., 502ₙ can transmit the gathered data to the mobilization modeling entity 504 where the mobilization modeling entity 504 can personalize a mobilization models for a particular user.

Additionally, users who opt-in to sharing their personal data, genetic data, and personalized mobilization information can transmit the information back to the mobilization modeling entity 504. The mobilization modeling entity 504 can also include a mobilization prediction model learning engine 506 that can use the crowd-sourced personal mobilization information from the distributed user base to refine the prediction models. Also, machine learning algorithms can be employed to refine mobilization prediction models based on the gathered user information, activity data, blood sample data, personal genetic data, etc.

In some cases, the mobilization modeling entity 504 can collect activity data, blood sample data, genetic data, etc. from a distributed set of user devices 502ₐ, 502_{b}, ..., 502ₙ and construct mobilization prediction models for the devices' users. However, in some cases, one or more of the user device's 502ₐ, 502_{b}, ..., 502ₙ can also store a local version of a clinically-derived mobilization prediction model and/ or can be configured with an application for retrieving a clinically-derived mobilization prediction model from a network based location (e.g. from the mobilization modeling entity 504). Similarly, one or more of the user device's 502ₐ, 502_{b}, ..., 502ₙ can also be configured with an application for using the clinically-derived mobilization prediction model, along with collected activity data, blood sample data, genetic data, etc. to construct a personalized mobilization prediction model. Likewise, one or more of the user device's 502ₐ, 502_{b}, .., 502ₙ can be configured to retrieve an application for creating a personalized mobilization prediction model from a network location (e.g. from the mobilization modeling entity 504, from a digital distribution platform for mobile apps, etc.)

As explained above, knowledge of the effects of activity on blood cell mobilization can be used to enhance the accuracy of a blood test. Additionally, in some cases, a clinically-derived blood cell mobilization prediction model and/ or the personalized blood cell mobilization prediction model can be used to prescribe an activity regimen to increase the accuracy of a blood test. For example, to determine a more accurate circulating cell count for an individual minus activity-related effects, short term and medium term effects of activity can be excluded. To accomplish the exclusion of activity-related effects, an activity regimen can be prescribed. For example, a prescription can recommend that an individual should not perform any strenuous exercise for forty-eight hours before a blood test and very little exercise for twenty-four hours leading up to the test. The prescription can also dictate that blood measurements are taken in a fully rested state, with no movement in the previous forty-five minutes.

Additionally, the activity regimen can be coupled with the use of telemetry to monitor quantifiable metrics (e.g. heart rate) and to receive self-reported data describing activity (e.g. manual input of exercise data, self-reported feelings of "soreness", etc.). For example, the activity regimen can also suggest that a heart rate monitor should be worn for at least two days prior to the blood sampling to help exclude the effect of rate of blood flow on the determination of circulating blood cells minus activity.

The use of telemetry can also be used to compare past activity and blood sample data with contemporary (i.e. around the time of a blood draw) data to allow comparative analysis. For example, recent heart rate history with an accelerometer will show how close to normal resting state the individual's heart rate was at the time of blood sampling. Also, selected population data can help compensate for the effect of accelerated heart rate (i.e. over normal resting state) on circulating cell concentration.

Also, experimental data shows that increases in circulating mature immune cells and stem/progenitor cells can vary significantly depending on the individual, their level of exertion and the length of exercise. Establishing baselines for each individual is therefore important for the proper generalization of white blood cell and stem cell mobilization over time for that individual. In some embodiments, a clinically-derived blood cell mobilization prediction model and/or personalized blood cell mobilization prediction model can be used, in connection with telemetry, to predict the concentration of white blood cells and stem cells in an individual over a period of time without the frequent need for drawing blood.

As explained above, knowledge of the effects of activity on blood cell mobilization can be utilized to mobilize specific cells known to combat specific illnesses and disorders, and can be used to increase the effectiveness of a treatment (e.g. a vaccine, chemotherapy) by priming an individual's blood and recommending post-treatment activity to further enhance the treatment. Accordingly, some embodiments of the present technology involves using a clinically-derived blood cell mobilization prediction model and/ or a personalized blood cell mobilization prediction model along with activity data collected from telemetry to optimize the effectiveness of a vaccine.

A personalized blood cell mobilization prediction model can be used to predict individual vaccine response based on baseline data and optimal or individually pre-set activity in pre-priming, priming, treatment, and post-treatment phases. An enhanced individual response can be achieved by eliciting an increase in the circulation and concentration of the immune cells which interact with antigens present in the vaccine.

In some cases, phasing can be scheduled as follows:
Phase (1) - Pre-Priming Phase: Forty-Eight Hours Prior to Six Hours Prior to Vaccination
Phase (2) - Priming Phase: Six Hours Prior to Vaccination Until the Time of the Vaccination
Phase (3) - Treatment Phase: Time of Vaccination
Phase (4) - Post-Treatment Enhancement Phase: Immediately Following Vaccination and Up to Six Hours Following Vaccination

Although specific time periods are listed herein, those with ordinary skill in the art having the benefit of the disclosure will readily appreciate that a wide variety of time periods can be prescribed to accomplish the goals of priming an individual to receive a vaccination, undergo a treatment, receive a medication, etc.

In some cases, the Pre-Priming Phase can include a period of approximately forty-eight hours preceding priming phase. In the Pre-Priming Phase an individual's activity can be tracked with a heartrate monitor, GPS sensor, accelerometer, etc. The activity data can also be processed locally on the activity tracking device, processed locally on another device in communication (e.g. Bluetooth link) with the activity tracking device, and transmitted to a monitoring entity to determine whether the activity data indicates deviation from a predetermined regimen. In the event that the activity data deviates to a threshold degree, a notification can be sent to the individual to correct a behavior.

In some cases, the Priming Phase can include a period of six hours prior to a scheduled treatment. In the Priming Phase, activity can be monitored and directed in a controlled fashion. For example, computer-assisted generation of a sustained alteration in immune system concentration and distribution can be performed through monitored activity where the alteration enhances the immediate capture and bio-processing of the vaccine or other treatment. The computer-assisted activity recommendation can include activity recommended by the computer program with real time feedback and can be based a clinically-derived blood cell mobilization prediction model and/ or personalized blood cell mobilization prediction model. In some cases, the computer may suggest activity or passively predict immune response during priming based on various biosensors including heart rate, motion, GPS, electrocardiogram (EKG), galvanic skin response etc.

In some cases, the Treatment Phase includes the administration of the vaccine or other treatment and can include immunotherapy/immunomodulatory therapy. Finally, the Post-Treatment enhancement phase can involve a limited window to use activity to further enhance vaccination response in part by maximization of immunosurveillance and increased communication between white blood cells. An additional limited, individual window can also be utilized to capitalize on sustained mobilization effect from the pre-priming and priming phases earlier in the day. In some cases, a diminishing weight is assigned to positive effect of exercise on vaccine as time passes in this Post Treatment phase.

In some cases, the timing and administration of the Phases are modified by a variety of variables including the type of vaccination tissue, the rate blood flow to injected tissue, the estimated relevant white blood cell surface protein concentrations, the different white blood cell types in blood, the type and duration of post treatment activity, and the time that vaccine takes to completely disseminate from injection site which can be influenced locally to the tissue and globally (e.g. muscle pump at vaccine site verses whole body cardiovascular workout).

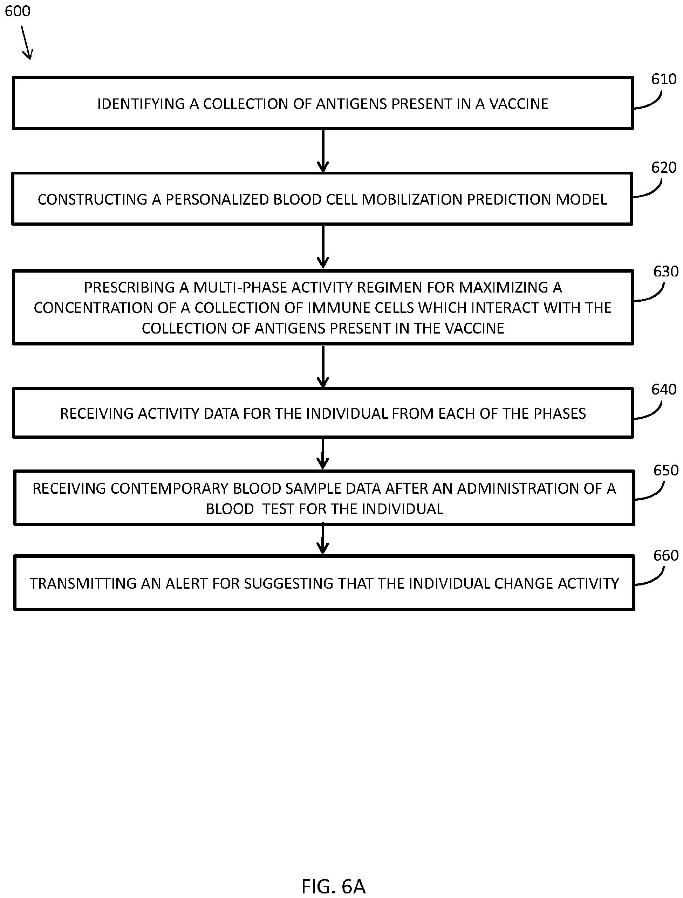

FIG. 6A illustrates a method 600 of optimizing a vaccine. The method 600 begins with identifying a collection of antigens present in a vaccine 610. Next, the method involves constructing a personalized blood cell mobilization prediction model 620 using a clinically-derived blood cell mobilization prediction model and a set of collected individual data.

Next, using the personalized blood cell mobilization prediction model and the collection of antigens present in a vaccine, the method 600 involves prescribing a multi-phase activity regimen for maximizing a concentration of a collection of immune cells which interact with the collection of antigens present in the vaccine 630. Also, the method 600 involves receiving activity data for the individual from each of the phases 640, receiving contemporary blood sample data after an administration of a blood test for the individual 650, and detecting, using the personalized blood cell mobilization prediction model, that the received activity data deviates from the activity regimen to a predetermined threshold degree.

Upon detecting the deviation away from the prescribed activity regimen, the method 600 involves transmitting, to a feedback device, an instruction that is effective to cause the feedback device to display an alert for suggesting that the individual change activity 660.

In some cases, the multi-phase activity regimen involves a pre-priming phase, a priming phase, a treatment phase, and a post-treatment enhancement phase. The pre-priming phase can encompass a period of time fifty-four hours prior to the vaccine and can involve telemetric tracking of heartrate data. The priming phase can encompass a six hour period prior to the vaccination and can involve a local, controlled, computer-assisted activity tracking. The treatment phase can include the time of the vaccination and can involve one or more of a immunotherapy therapy technique and an immunomodulatory therapy technique. Also, the post-treatment enhancement phase can encompass a six hour period after the treatment and can involve additional telemetric tracking of heartrate data and transmission of an instruction to a user device that is effective to cause the user device to display an alert for suggesting that the individual change activity upon detecting, using the personalized blood cell mobilization prediction model, that the heartrate data deviates from a suggested heartrate to a predetermined threshold degree.

Experimental data confirms that a prescribed exercise regimen optimizes the effects of a vaccine. Table 1 shows a comparison of Hepatitis B Antigen positive cells (HBsAg+) per one million Peripheral Blood Mononuclear Cells (PBMC) fourteen data after participants receive a Hepatitis B vaccination.

**Table 1**

| **HBsAg+ cells/1M PBMC** | | |
|---|---|---|
| | Control | Exercise |
| | 17 | 42 |
| | 13 | 85 |
| | | 96 |
| | | 75 |
| **Mean** | **IS** | **74** |
| SEM | 1.6 | 11.7 |
| SEM-top | 16.5 | 86.2 |
| SEM-bot | 13.3 | 62.7 |
| p-value | 0.028 | |

The Exercise/activity group from Table 1 exercised vigorously during priming and post-vaccination phases while the control group refrained from vigorous exercise on vaccination day. Table 2 shows vaccination response described in Kinetics of Hepatitis B Surface Antigen-Specific Immune Responses in Acute and Chronic Hepatitis B or After HBs Vaccination: Stimulation of the In Vitro Antibody Response by Interferon Gamma, Bocher et al., 1999 following a single Hepatitis B vaccination at day 14.

**Table 2**

| Bocher et al 1999 | |
|---|---|
| **Mean** | **34** |
| SEM-bot | 18.5 |
| SEM-top | 49.5 |

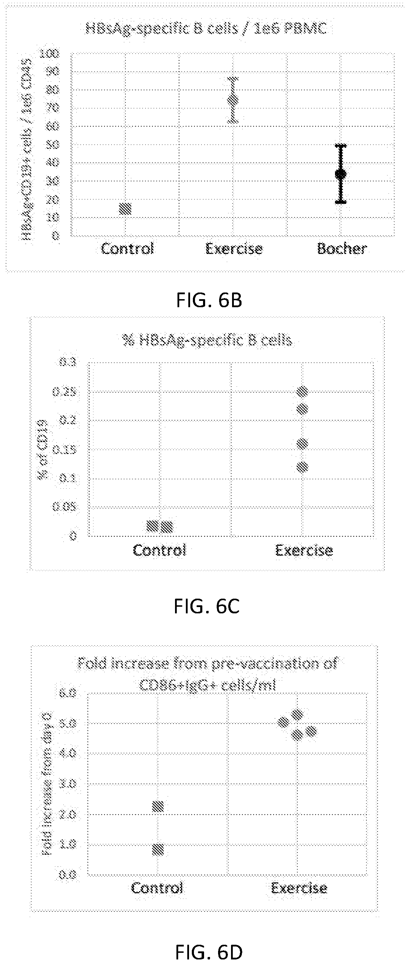

FIG. 6B illustrates the results of an experiment comparing Hepatitis B Antigen positive cells (HBsAg+) per one million Peripheral Blood Mononuclear Cells (PBMC) in the Exercise Group vs. the Control Group vs. Bocker et al. FIG. 6C illustrates the results of an experiment comparing a percentage of Hepatitis B Antigen positive cells (HBsAg+) in blood for the Exercise Group vs. the Control Group. FIG. 6D illustrates a fold increase in the results of an experiment comparing a percentage of Hepatitis B Antigen positive cells (HBsAg+).

As explained above, the use of telemetric devices allows for the collection of activity data (e.g. heart rate data) that can be used to construct personalized blood cell mobilization prediction model, monitor activity data, etc. However, device malfunction and/ or user error can significantly jeopardize the availability and the reliability of data collected from telemetric devices. Therefore, some embodiments of the present technology involve accounting for unavailability of telemetric data, inconsistency in telemetric data, etc.

In some cases, the measurements from hardware appearing to indicate highly abnormal heart rate, movement speed, acceleration, temperature, etc. can be characterized as a malfunction. This can be due to actual malfunction of the sensor, or simply removal of the sensor from its appropriate location (e.g. removal of watch-based heart rate monitor from wrist). Characterizing the abnormalities as a malfunction can prevent both inaccurate predictions for an individual user and also inaccurate data being added to the prediction databases. However, since the individual's activity that takes place when the activity data that is disregarded as being unreliable is still an important input in prediction, some embodiments of the present technology involve compensating for the malfunctions. For example, software in the activity tracking device or in the modeling entity can automatically adjust compensate for a sensor malfunction based on other sensor readings and context. For example, inputs from separate sensor units can be combined to cross-validate readings and compensate for missing data. In one specific example, when an individual's heart rate monitor watch is removed or malfunctioning, a GPS device and accelerometer (e.g. in a smartphone) can determine a level of activity of the individual. Data from the accelerometer, such as angle of phone, and movement patterns, help to determine if unit is physically on the user or also unable to take measurements in the timeframe that watch is unable to contribute to prediction model.

In some cases, automatic functional restoration calculations can involve using compensating factors such as time of day of sensor removal, activity levels throughout the day and the specific location of the user at time of removal and restoration of sensors. Additionally, a software application can prompt the user to self-report activity or exertion levels from during period of sensor malfunction to assist in prediction functionality and confidence in calculations.

In some cases, output resolution of the prediction can be reduced to compensate for uncertainty of predictions. For example, only short term mobilization results may be displayed until system gains confidence on medium term and long term mobilizations and baselines. Additionally, the mobilization results that are displayed and recorded can be of different resolutions. For instance, the user interface can display only generalized relative mobilization values, whereas the application may record a predicted mobilization percentage with a confidence interval.

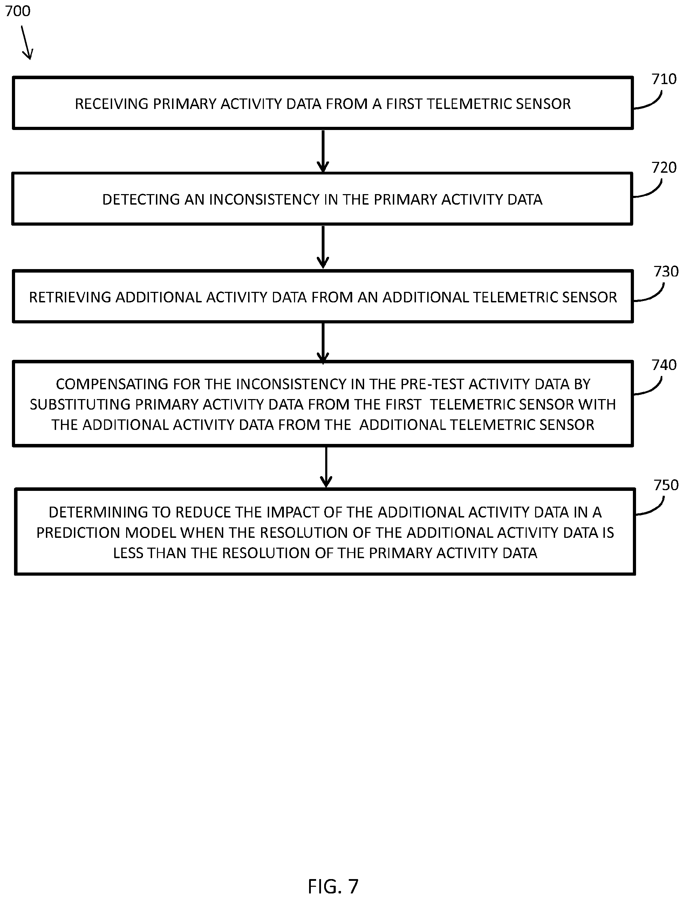

FIG. 7 illustrates a method 700 of automatically compensating for sensor malfunctions. The method 700 involves receiving primary activity data from a first telemetric sensor 710. For example, the primary activity data can be data that is from a source that is typically highly reliable in indicating an individual's activity level, e.g. data from a wearable heart rate monitor. Next, the method 700 can involve detecting an inconsistency in the primary activity data 720 and retrieving additional activity data from an additional telemetric sensor 730. In some cases, the additional activity data can be retrieved from a source that is less reliable than the source of the primary activity data. For example, the additional activity data can include GPS data from a smartphone and timestamp information that can be used to construct a dataset describing an individual's movements, distance traveled velocity, etc. Although the example data is indicative of activity, it can be less reliable (i.e. lower resolution) since the actual exertion (otherwise measured by heartrate) is more difficult to quantify, e.g. since the individual might have been running, riding a bicycle, etc.

Next, the method 700 involves compensating for the inconsistency in the pre-test activity data by substituting primary activity data from the first telemetric sensor with the additional activity data from the additional telemetric sensor 740. Also, the method 700 can involve determining to reduce the impact of the additional activity data in a prediction model when the resolution of the additional activity data is less than the resolution of the primary activity data 750.

In some cases, sensor malfunction, individual non-compliance proper sensor placement, etc. can cause gaps in telemetric data. Therefore, some embodiments of the present technology involve substituting missing data with either an average for time of day or based on extrapolation of telemetry preceding and following gap.

As described above, one aspect of the present technology is the gathering and use of data available from various sources. The present disclosure contemplates that in some instances, this gathered data may include personal information data that uniquely identifies or can be used to contact or locate a specific person. Such personal information data can include demographic data, location-based data, telephone numbers, email addresses, twitter ID's, home addresses, or any other identifying information.

The present disclosure further contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure. For example, personal information from users should be collected for legitimate and reasonable uses of the entity and not shared or sold outside of those legitimate uses. Further, such collection should occur only after receiving the informed consent of the users. Additionally, such entities would take any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of advertisement delivery services, the present technology can be configured to allow users to select to "opt in" or "opt out" of participation in the collection of personal information data during registration for services.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, content can be selected and delivered to users by inferring preferences based on non-personal information data or a bare minimum amount of personal information, such as the content being requested by the device associated with a user, other non-personal information available to the content delivery services, or publically available information.

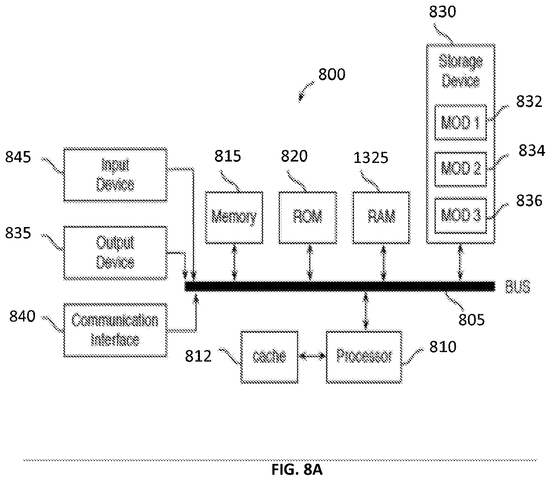

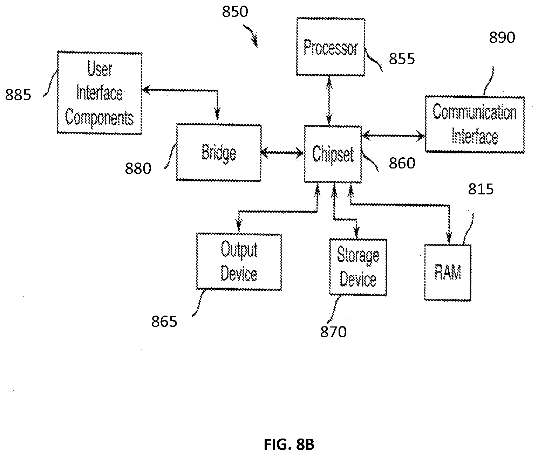

FIG. 8A and FIG. 8B illustrate exemplary possible system embodiments. The more appropriate embodiment will be apparent to those of ordinary skill in the art when practicing the present technology. Persons of ordinary skill in the art will also readily appreciate that other system embodiments are possible.

FIG. 8A illustrates a conventional system bus computing system architecture 800 wherein the components of the system are in electrical communication with each other using a bus 805. Exemplary system 800 includes a processing unit (CPU or processor) 810 and a system bus 805 that couples various system components including the system memory 815, such as read only memory (ROM) 820 and random access memory (RAM) 825, to the processor 810. The system 800 can include a cache of high-speed memory connected directly with, in close proximity to, or integrated as part of the processor 810. The system 800 can copy data from the memory 815 and/or the storage device 830 to the cache 812 for quick access by the processor 810. In this way, the cache can provide a performance boost that avoids processor 810 delays while waiting for data. These and other modules can control or be configured to control the processor 810 to perform various actions. Other system memory 815 may be available for use as well. The memory 815 can include multiple different types of memory with different performance characteristics. The processor 810 can include any general purpose processor and a hardware module or software module, such as module 1 832, module 2 834, and module 3 836 stored in storage device 830, configured to control the processor 810 as well as a special-purpose processor where software instructions are incorporated into the actual processor design. The processor 810 may essentially be a completely self-contained computing system, containing multiple cores or processors, a bus, memory controller, cache, etc. A multi-core processor may be symmetric or asymmetric.

To enable user interaction with the computing device 800, an input device 845 can represent any number of input mechanisms, such as a microphone for speech, a touch-sensitive screen for gesture or graphical input, keyboard, mouse, motion input, speech and so forth. An output device 835 can also be one or more of a number of output mechanisms known to those of skill in the art. In some instances, multimodal systems can enable a user to provide multiple types of input to communicate with the computing device 800. The communications interface 840 can generally govern and manage the user input and system output. There is no restriction on operating on any particular hardware arrangement and therefore the basic features here may easily be substituted for improved hardware or firmware arrangements as they are developed.

Storage device 830 is a non-volatile memory and can be a hard disk or other types of computer readable media which can store data that are accessible by a computer, such as magnetic cassettes, flash memory cards, solid state memory devices, digital versatile disks, cartridges, random access memories (RAMs) 825, read only memory (ROM) 820, and hybrids thereof.

The storage device 830 can include software modules 832, 834, 836 for controlling the processor 810. Other hardware or software modules are contemplated. The storage device 830 can be connected to the system bus 805. In one aspect, a hardware module that performs a particular function can include the software component stored in a computer-readable medium in connection with the necessary hardware components, such as the processor 810, bus 805, display 835, and so forth, to carry out the function.

FIG. 8B illustrates a computer system 850 having a chipset architecture that can be used in executing the described method and generating and displaying a graphical user interface (GUI). Computer system 850 is an example of computer hardware, software, and firmware that can be used to implement the disclosed technology. System 850 can include a processor 855, representative of any number of physically and/or logically distinct resources capable of executing software, firmware, and hardware configured to perform identified computations. Processor 855 can communicate with a chipset 860 that can control input to and output from processor 855. In this example, chipset 860 outputs information to output 865, such as a display, and can read and write information to storage device 870, which can include magnetic media, and solid state media, for example. Chipset 860 can also read data from and write data to RAM 875. A bridge 880 for interfacing with a variety of user interface components 885 can be provided for interfacing with chipset 860. Such user interface components 885 can include a keyboard, a microphone, touch detection and processing circuitry, a pointing device, such as a mouse, and so on. In general, inputs to system 850 can come from any of a variety of sources, machine generated and/or human generated.

Chipset 860 can also interface with one or more communication interfaces 890 that can have different physical interfaces. Such communication interfaces can include interfaces for wired and wireless local area networks, for broadband wireless networks, as well as personal area networks. Some applications of the methods for generating, displaying, and using the GUI disclosed herein can include receiving ordered datasets over the physical interface or be generated by the machine itself by processor 855 analyzing data stored in storage 870 or 875. Further, the machine can receive inputs from a user via user interface components 885 and execute appropriate functions, such as browsing functions by interpreting these inputs using processor 855.

It can be appreciated that exemplary systems 800 and 850 can have more than one processor 810 or be part of a group or cluster of computing devices networked together to provide greater processing capability.

For clarity of explanation, in some instances the present technology may be presented as including individual functional blocks including functional blocks comprising devices, device components, steps or routines in a method embodied in software, or combinations of hardware and software.

In some embodiments the computer-readable storage devices, mediums, and memories can include a cable or wireless signal containing a bit stream and the like. However, when mentioned, non-transitory computer-readable storage media expressly exclude media such as energy, carrier signals, electromagnetic waves, and signals per se.

Methods according to the above-described examples can be implemented using computer-executable instructions that are stored or otherwise available from computer readable media. Such instructions can comprise, for example, instructions and data which cause or otherwise configure a general purpose computer, special purpose computer, or special purpose processing device to perform a certain function or group of functions. Portions of computer resources used can be accessible over a network. The computer executable instructions may be, for example, binaries, intermediate format instructions such as assembly language, firmware, or source code. Examples of computer-readable media that may be used to store instructions, information used, and/or information created during methods according to described examples include magnetic or optical disks, flash memory, USB devices provided with non-volatile memory, networked storage devices, and so on.

Devices implementing methods according to these disclosures can comprise hardware, firmware and/or software, and can take any of a variety of form factors. Typical examples of such form factors include laptops, smart phones, small form factor personal computers, personal digital assistants, and so on. Functionality described herein also can be embodied in peripherals or add-in cards. Such functionality can also be implemented on a circuit board among different chips or different processes executing in a single device, by way of further example.

The instructions, media for conveying such instructions, computing resources for executing them, and other structures for supporting such computing resources are means for providing the functions described in these disclosures.

Although a variety of examples and other information was used, no limitation of the claims should be implied based on particular features or arrangements in such examples, as one of ordinary skill would be able to use these examples to derive a wide variety of implementations. Further and although some subject matter may have been described in language specific to examples of structural features and/or method steps, it is to be understood that the subject matter defined in the appended claims is defining the scope of the present invention. For example, such functionality can be distributed differently or performed in components other than those identified herein. Rather, the described features and steps are disclosed as examples of components of systems.

## Claims

1. A system for enhancing the results of a blood test (500) comprising:
a processor (810); and
a computer-readable storage medium (830) having stored therein instructions which, when executed by the processor (810), cause the processor to perform operations comprising:
constructing, for an individual, a personalized blood cell mobilization prediction model (440) using a clinically-derived blood cell mobilization prediction model (410) and a set of collected individual data (420);
receiving pre-test activity data for the individual (450);
receiving contemporary blood sample data after an administration of a blood test for the individual (460);
predicting a contemporary effect of the pre-test activity data by applying the pre-test activity data to the personalized blood cell mobilization prediction model (470); and
creating an enhanced blood panel by excluding the contemporary effect of the contemporary activity from the contemporary blood sample data (480).

2. The system of claim 1, wherein the instructions further cause the processor to construct the clinically-derived blood cell mobilization prediction model (200) by performing the steps of:
collecting, from each participant in a statistical sample of participants, blood sample data at a plurality of times over a period (240);
collecting, from each participant in the statistical sample of participants, personal data relating to blood cell mobilization response (210);
collecting, from each participant in the statistical sample of participants, genetic data relating to blood cell mobilization response (220);
collecting over the period, from each participant in the statistical sample of participants, activity data (230); and
analyzing the blood sample data to correlate one or more changes in mobilization rates for one or more blood cells and one or more changes to blood cell surface features to one or more factors selected from the personal data (250), the genetic data, and the activity data.

3. The system of claim 1, wherein the instructions further cause the processor to perform the steps of collecting, from the individual, personal data relating to blood cell mobilization response (320) and genetic data relating to blood cell mobilization response (330).

4. The system of claim 3, wherein constructing the personalized blood cell mobilization prediction model (370) further comprises:
collecting a set of historical blood sample data for the individual (350), and historical activity data for the individual (340);
analyzing the historical blood sample data and the historical activity data for the individual; and
determining whether to validate or invalidate one or more correlations in the clinically-derived blood cell mobilization prediction model relating to one or more changes in mobilization rates for one or more blood cells and one or more changes to blood cell surface features to one or more factors selected from personal data, genetic data, and activity data based on one or more actual changes in mobilization rates for one or more blood cells and one or more actual changes to blood cell surface features observed in the historical blood sample data (360).

5. The system of claim 1, wherein the instructions further cause the processor to perform the steps of:
inspecting the pre-test activity data for the individual;
detecting an inconsistency in the pre-test activity data (720);
receiving additional pre-test activity data from an additional telemetric sensor (730); and
substituting the pre-test activity data from the telemetric sensor with the additional pre-test activity data from the additional telemetric sensor (740).

6. The system of claim 5, wherein the inconsistency comprises a loss of connectivity of a telemetric sensor for gathering pre-test activity data.

7. The system of claim 5, wherein the instructions further cause the processor to perform the steps of:
reducing an impact of the additional activity data in predicting a contemporary effect when a resolution of the additional pre-test activity data is less than a resolution of the pre-test activity data (750).

8. The system of claim 5, wherein the telemetric sensor comprises a heartrate monitor, wherein the additional telemetric sensor comprises an accelerometer, and wherein the additional pre-test activity data comprises a predicted heart rate dataset derived from motion data collected from the accelerometer.

9. The system of claim 1, wherein the pre-test activity data is collected from a telemetric sensor in an activity tracking device worn by the individual, and wherein the instructions further cause the processor to perform the steps of:
wirelessly receiving, from the telemetric sensor, a stream of real-time pre-test activity data;
detecting, using the personalized blood cell mobilization prediction model, that the real-time pre-test activity data will cause a contemporary effect that will negatively affect the accuracy of the enhanced blood panel to a predetermined threshold degree; and
wirelessly transmitting, to the activity tracking device, an instruction that is effective to cause the activity tracking device to display an alert for suggesting that the individual change activity.

10. A non-transitory computer-readable storage medium (830) having stored therein instructions which, when executed by a processor, cause the processor to perform operations comprising:
constructing, for an individual, a personalized blood cell mobilization prediction model (440) using a clinically-derived blood cell mobilization prediction model (410) and a set of collected individual data (420);
receiving pre-test activity data for the individual (450);
receiving contemporary blood sample data after an administration of a blood test for the individual (460);
predicting a contemporary effect of the pre-test activity data by applying the pre-test activity data to the personalized blood cell mobilization prediction model (470); and
creating an enhanced blood panel by excluding the contemporary effect of the contemporary activity from the contemporary blood sample data (480).

11. The non-transitory computer-readable storage medium of claim 10, wherein the instructions further cause the processor to perform the steps of collecting, from the individual, personal data relating to blood cell mobilization response (210) and genetic data relating to blood cell mobilization response (220).

12. The non-transitory computer-readable storage medium of claim 11, wherein constructing the personalized blood cell mobilization prediction model further comprises:
collecting a set of historical blood sample data for the individual, and historical activity data for the individual (340);
analyzing the historical blood sample data and the historical activity data for the individual; and
determining whether to validate or invalidate one or more correlations in the clinically-derived blood cell mobilization prediction model relating to one or more changes in mobilization rates for one or more blood cells and one or more changes to blood cell surface features to one or more factors selected from personal data, genetic data, and activity data based on one or more actual changes in mobilization rates for one or more blood cells and one or more actual changes to blood cell surface features observed in the historical blood sample data (360).

13. The non-transitory computer-readable storage medium of claim 10, wherein the instructions further cause the processor to perform the steps of:
inspecting the pre-test activity data for the individual;
detecting an inconsistency in the pre-test activity data (720);
receiving additional pre-test activity data from an additional telemetric sensor (730); and
substituting the pre-test activity data from the telemetric sensor with the additional pre-test activity data from the additional telemetric sensor (740).

## Patentansprüche

1. System zur Verbesserung der Ergebnisse einer Blutuntersuchung (500), umfassend:
einen Prozessor (810) und
ein computerlesbares Speichermedium (830) mit darauf gespeicherten Anweisungen, die, wenn sie vom Prozessor (810) ausgeführt werden, den Prozessor veranlassen, Operationen auszuführen, umfassend:
Erstellen eines personalisierten Blutzellenmobilisierungs-Vorhersagemodells (440) für ein Individuum unter Verwendung eines klinisch gewonnenen Blutzellenmobilisierungs-Vorhersagemodells (410) und eines Satzes gesammelter individueller Daten (420),
Empfangen von Vor-Test-Aktivitätsdaten für das Individuum (450),
Empfangen zeitnaher Blutprobendaten nach Durchführung einer Blutuntersuchung für das Individuum (460),
Vorhersagen einer zeitnahen Wirkung der Vor-Test-Aktivitätsdaten durch Anwenden der Vor-Test-Aktivitätsdaten auf das personalisierte Blutzellenmobilisierungs-Vorhersagemodell (470) und
Schaffung eines verbesserten Blutbilds durch Ausschluss der zeitnahen Wirkung der zeitnahen Aktivität aus den zeitnahen Blutprobendaten (480).

2. System nach Anspruch 1, wobei die Anweisungen den Prozessor ferner veranlassen, das klinisch gewonnene Blutzellenmobilisierungs-Vorhersagemodell zu erstellen (200) durch Ausführen der Schritte:
Sammeln von Blutprobendaten von jedem Teilnehmer in einer statistischen Stichprobe von Teilnehmern zu mehreren Zeitpunkten über einen Zeitraum (240),
Sammeln persönlicher Daten von jedem Teilnehmer in der statistischen Stichprobe von Teilnehmern in Bezug auf die Blutzellenmobilisierungsreaktion (210),
Sammeln genetischer Daten von jedem Teilnehmer in der statistischen Stichprobe von Teilnehmern, die sich auf die Blutzellenmobilisierungsreaktion beziehen (220),
Sammeln von Aktivitätsdaten von jedem Teilnehmer in der statistischen Stichprobe der Teilnehmer über den Zeitraum (230) und
Analysieren der Blutprobendaten, um eine oder mehrere Änderungen der Mobilisierungsraten für eine oder mehrere Blutzellen und eine oder mehrere Änderungen der Oberflächenmerkmale der Blutzellen mit einem oder mehreren Faktoren zu korrelieren, die ausgewählt sind aus den persönlichen Daten, den genetischen Daten und den Aktivitätsdaten (250).

3. System nach Anspruch 1, wobei die Anweisungen den Prozessor ferner veranlassen, die Schritte des Sammelns persönlicher Daten in Bezug auf die Blutzellenmobilisierungsreaktion (320) und genetischer Daten (330), die sich auf die Blutzellenmobilisierungsreaktion beziehen, von dem Individuum auszuführen.

4. System nach Anspruch 3, wobei das Erstellen des personalisierten Blutzellenmobilisierungs-Vorhersagemodells (370) ferner umfasst:
Sammeln eines Satzes historischer Blutprobendaten für das Individuum (350) und historischer Aktivitätsdaten für das Individuum (340),
Analysieren der historischen Blutprobendaten und der historischen Aktivitätsdaten für das Individuum und
Bestimmen, ob eine oder mehrere Korrelationen in dem klinisch gewonnenen Blutzellenmobilisierungs-Vorhersagemodell in Bezug auf eine oder mehrere Änderungen der Mobilisierungsraten für eine oder mehrere Blutzellen und eine oder mehrere Änderungen der Oberflächenmerkmale der Blutzellen mit einem oder mehreren Faktoren, ausgewählt aus persönlichen Daten, genetischen Daten und Aktivitätsdaten, zu bestätigen oder zu entkräften sind, basierend auf einer oder mehreren tatsächlichen Änderungen der Mobilisierungsraten für eine oder mehrere Blutzellen und einer oder mehreren tatsächlichen Änderungen der Oberflächenmerkmale der Blutzellen, die in den historischen Blutprobendaten beobachtet wurden (360).

5. System nach Anspruch 1, wobei die Anweisungen den Prozessor ferner veranlassen, die Schritte auszuführen:
Prüfen der Vor-Test-Aktivitätsdaten für das Individuum,
Erkennen einer Inkonsistenz in den Vor-Test-Aktivitätsdaten (720),
Empfangen zusätzlicher Vor-Test-Aktivitätsdaten von einem zusätzlichen Telemetriesensor (730) und
Ersetzen der Vor-Test-Aktivitätsdaten von dem Telemetriesensor durch die zusätzlichen Vor-Test-Aktivitätsdaten von dem zusätzlichen Telemetriesensor (740).

6. System nach Anspruch 5, wobei die Inkonsistenz einen Konnektivitätsverlust eines Telemetriesensors zum Sammeln von Vor-Test-Aktivitätsdaten umfasst.

7. System nach Anspruch 5, wobei die Anweisungen den Prozessor ferner veranlassen, die Schritte auszuführen:
Reduzieren eines Einflusses der zusätzlichen Aktivitätsdaten bei der Vorhersage einer zeitnahen Wirkung, wenn eine Auflösung der zusätzlichen Vor-Test-Aktivitätsdaten geringer ist als eine Auflösung der Vor-Test-Aktivitätsdaten (750).

8. System nach Anspruch 5, wobei der Telemetriesensor einen Herzfrequenzmonitor umfasst, wobei der zusätzliche Telemetriesensor einen Beschleunigungsmesser umfasst und wobei die zusätzlichen Vor-Test-Aktivitätsdaten einen vorhergesagten Herzfrequenzdatensatz umfassen, der aus vom Beschleunigungsmesser gesammelten Bewegungsdaten abgeleitet ist.

9. System nach Anspruch 1, wobei die Vor-Test-Aktivitätsdaten von einem Telemetriesensor in einem von der Person getragenen Aktivitätsverfolgungsgerät gesammelt werden und wobei die Anweisungen den Prozessor ferner veranlassen, die Schritte auszuführen:
drahtloses Empfangen eines Stroms von Echtzeit-Vor-Test-Aktivitätsdaten von dem Telemetriesensor,
Erkennen unter Verwendung des personalisierten Blutzellenmobilisierungs-Vorhersagemodells, dass die Echtzeit-Vor-Test-Aktivitätsdaten eine zeitnahe Wirkung verursachen werden, die die Genauigkeit des verbesserten Blutbilds bis zu einem vorgegebenen Schwellenwertgrad negativ beeinflussen wird, und
drahtloses Senden einer Anweisung an das Aktivitätsverfolgungsgerät, die dazu führt, das Aktivitätsverfolgungsgerät zu veranlassen, eine Warnung anzuzeigen, die dem Individuum eine Aktivitätsänderung vorschlägt.

10. Nichtflüchtiges computerlesbares Speichermedium (830) mit darauf gespeicherten Anweisungen, die, wenn sie von einem Prozessor ausgeführt werden, den Prozessor veranlassen, Operationen auszuführen, umfassend:
Erstellen eines personalisierten Blutzellenmobilisierungs-Vorhersagemodells (440) für ein Individuum unter Verwendung eines klinisch gewonnenen Blutzellenmobilisierungs-Vorhersagemodells (410) und eines Satzes gesammelter individueller Daten (420),
Empfangen von Vor-Test-Aktivitätsdaten für das Individuum (450),
Empfangen zeitnaher Blutprobendaten nach einer Durchführung einer Blutuntersuchung für das Individuum (460),
Vorhersagen einer zeitnahen Wirkung der Vor-Test-Aktivitätsdaten durch Anwenden der Vor-Test-Aktivitätsdaten auf das personalisierte Blutzellenmobilisierungs-Vorhersagemodell (470) und
Schaffen eines verbesserten Blutbilds durch Ausschluss der zeitnahen Wirkung der zeitnahen Aktivität aus den zeitnahen Blutprobendaten (480).

11. Nichtflüchtiges computerlesbares Speichermedium nach Anspruch 10, wobei die Anweisungen den Prozessor ferner veranlassen, die Schritte des Sammelns persönlicher Daten in Bezug auf die Blutzellenmobilisierungsreaktion (210) und genetischer Daten, die sich auf die Blutzellenmobilisierungsreaktion beziehen (220), von dem Individuum auszuführen.

12. Nichtflüchtiges computerlesbares Speichermedium nach Anspruch 11, wobei das Erstellen des personalisierten Blutzellenmobilisierungs-Vorhersagemodells ferner umfasst:
Sammeln eines Satzes historischer Blutprobendaten für das Individuum und historischer Aktivitätsdaten für das Individuum (340),
Analysieren der historischen Blutprobendaten und der historischen Aktivitätsdaten für das Individuum und
Bestimmen, ob eine oder mehrere Korrelationen in dem klinisch gewonnenen Blutzellenmobilisierungs-Vorhersagemodell in Bezug auf eine oder mehrere Änderungen der Mobilisierungsraten für eine oder mehrere Blutzellen und eine oder mehrere Änderungen der Oberflächenmerkmale der Blutzellen mit einem oder mehreren Faktoren, ausgewählt aus persönlichen Daten, genetischen Daten und Aktivitätsdaten, zu bestätigen oder zu entkräften sind, basierend auf einer oder mehreren tatsächlichen Änderungen der Mobilisierungsraten für eine oder mehrere Blutzellen und einer oder mehreren tatsächlichen Änderungen der Oberflächenmerkmale der Blutzellen, die in den historischen Blutprobendaten beobachtet wurden (360).

13. Nichtflüchtiges computerlesbares Speichermedium nach Anspruch 10, wobei die Anweisungen den Prozessor ferner veranlassen, die Schritte auszuführen:
Prüfen der Vor-Test-Aktivitätsdaten für das Individuum,
Erkennen einer Inkonsistenz in den Vor-Test-Aktivitätsdaten (720),
Empfangen zusätzlicher Vor-Test-Aktivitätsdaten von einem zusätzlichen Telemetriesensor (730) und
Ersetzen der Vor-Test-Aktivitätsdaten von dem Telemetriesensor durch die zusätzlichen Vor-Test-Aktivitätsdaten von dem zusätzlichen Telemetriesensor (740).

## Revendications

1. Système pour améliorer les résultats d'un test sanguin (500) comprenant :
un processeur (810) ; et
un support de stockage lisible par ordinateur (830) sur lequel sont stockées des instructions qui, lorsqu'elles sont exécutées par le processeur (810), amènent le processeur à effectuer des opérations comprenant :
la construction, pour un individu, d'un modèle de prédiction de mobilisation personnalisé des cellules sanguines (440) en utilisant un modèle de prédiction de mobilisation des cellules sanguines dérivé cliniquement (410) et un ensemble de données de l'individu collectées (420) ;
la réception de données d'activité de pré-test pour l'individu (450) ;
la réception de données d'échantillons de sang contemporaines après l'administration d'un test sanguin pour l'individu (460) ;
la prédiction d'un effet contemporain des données d'activité de pré-test en appliquant les données d'activité de pré-test au modèle de prédiction de mobilisation personnalisé des cellules sanguines (470) ; et
la création d'un panel sanguin amélioré en excluant l'effet contemporain de l'activité contemporaine des données de l'échantillon de sang contemporain (480).

2. Système selon la revendication 1, dans lequel les instructions amènent en outre le processeur à construire le modèle de prédiction de mobilisation des cellules sanguines dérivé cliniquement (200) en exécutant les étapes de :
collecte, à partir de chaque participant dans un échantillon statistique de participants, de données d'échantillons sanguins une pluralité de fois sur une période (240) ;
collecte, auprès de chaque participant dans l'échantillon statistique de participants, de données personnelles relatives à la réponse de mobilisation des cellules sanguines (210) ;
collecte, auprès de chaque participant de l'échantillon statistique de participants, de données génétiques relatives à la réponse de mobilisation des cellules sanguines (220) ;
collecte sur la période, auprès de chaque participant de l'échantillon statistique de participants, de données d'activité (230) ; et
analyse des données de l'échantillon de sang pour mettre en corrélation un ou plusieurs changements dans les taux de mobilisation pour une ou plusieurs cellules sanguines et une ou plusieurs modifications des caractéristiques de la surface des cellules sanguines avec un ou plusieurs facteurs sélectionnés parmi les données personnelles (250), les données génétiques et les données d'activité.

3. Système selon la revendication 1, dans lequel les instructions amènent en outre le processeur à effectuer les étapes de collecte, à partir de l'individu, de données personnelles relatives à la réponse de mobilisation des cellules sanguines (320) et de données génétiques relatives à la réponse de mobilisation des cellules sanguines (330).

4. Système selon la revendication 3, dans lequel la construction du modèle de prédiction de mobilisation personnalisé des cellules sanguines (370) comprend en outre :
la collecte d'un ensemble de données historiques d'échantillons de sang pour l'individu (350), et de données historiques d'activité pour l'individu (340) ;
l'analyse des données historiques d'échantillons de sang et des données historiques d'activité pour l'individu ; et
la détermination s'il faut valider ou invalider une ou plusieurs corrélations dans le modèle de prédiction de mobilisation des cellules sanguines dérivé cliniquement concernant un ou plusieurs changements des taux de mobilisation pour une ou plusieurs cellules sanguines et une ou plusieurs modifications des caractéristiques de surface des cellules sanguines à un ou plusieurs facteurs sélectionnés parmi les données personnelles, les données génétiques et les données d'activité basées sur un ou plusieurs changements réels des taux de mobilisation pour une ou plusieurs cellules sanguines et un ou plusieurs changements réels des caractéristiques de surface des cellules sanguines observés dans les données historiques de l'échantillon de sang (360).

5. Système selon la revendication 1, dans lequel les instructions amènent en outre le processeur à exécuter les étapes de :
inspection des données d'activité de pré-test pour l'individu ;
détection d'une incohérence dans les données d'activité de pré-test (720) ;
réception des données d'activité de pré-test supplémentaires depuis un capteur télémétrique supplémentaire (730) ; et
remplacement des données d'activité de pré-test en provenance du capteur télémétrique par les données d'activité de pré-test supplémentaires en provenance du capteur télémétrique supplémentaire (740).

6. Système selon la revendication 5, dans lequel l'incohérence comprend une perte de connectivité d'un capteur télémétrique pour recueillir des données d'activité de pré-test.

7. Système selon la revendication 5, dans lequel les instructions amènent en outre le processeur à exécuter les étapes de :
réduction d'un impact des données d'activité supplémentaires en prédisant un effet contemporain lorsqu'une résolution des données d'activité de pré-test supplémentaires est inférieure à une résolution des données d'activité de pré-test (750).

8. Système selon la revendication 5, dans lequel le capteur télémétrique comprend un moniteur de fréquence cardiaque, dans lequel le capteur télémétrique supplémentaire comprend un accéléromètre, et dans lequel les données d'activité de pré-test supplémentaires comprennent un ensemble de données de fréquence cardiaque prédit dérivé de données de mouvement collectées à partir de l'accéléromètre.

9. Système selon la revendication 1, dans lequel les données d'activité de pré-test sont collectées à partir d'un capteur télémétrique dans un dispositif de suivi d'activité porté par l'individu, et dans lequel les instructions amènent en outre le processeur à effectuer les étapes de :
réception sans fil, à partir du capteur télémétrique, d'un flux de données d'activité de pré-test en temps réel ;
détection, en utilisant le modèle de prédiction de mobilisation personnalisé des cellules sanguines, que les données d'activité de pré-test en temps réel provoqueront un effet contemporain qui affectera négativement la précision du panel sanguin amélioré à un degré seuil prédéterminé ; et
transmission sans fil, au dispositif de suivi d'activité, d'une instruction qui est efficace pour amener le dispositif de suivi d'activité à afficher une alerte pour suggérer que l'individu change d'activité.

10. Support de stockage non transitoire lisible par ordinateur (830) sur lequel sont stockées des instructions qui, lorsqu'elles sont exécutées par un processeur, amènent le processeur à effectuer des opérations comprenant :
la construction, pour un individu, d'un modèle de prédiction de mobilisation de cellules sanguines personnalisé (440) en utilisant un modèle de prédiction de mobilisation de cellules sanguines dérivé cliniquement (410) et un ensemble de données de l'individu collectées (420) ;
la réception de données d'activité de pré-test pour l'individu (450) ;
la réception de données d'échantillons de sang contemporaines après l'administration d'un test sanguin pour l'individu (460) ;
la prédiction d'un effet contemporain des données d'activité de pré-test en appliquant les données d'activité de pré-test au modèle de prédiction de mobilisation personnalisé des cellules sanguines (470) ; et
la création d'un panel sanguin amélioré en excluant l'effet contemporain de l'activité contemporaine des données de l'échantillon de sang contemporain (480).

11. Support de stockage non transitoire lisible par ordinateur selon la revendication 10, dans lequel les instructions amènent en outre le processeur à effectuer les étapes de collecte, à partir de l'individu, de données personnelles relatives à la réponse de mobilisation des cellules sanguines (210) et de données génétiques relatives à la réponse de mobilisation des cellules sanguines (220).

12. Support de stockage non transitoire lisible par ordinateur selon la revendication 11, dans lequel la construction du modèle de prédiction de mobilisation personnalisé des cellules sanguines comprend en outre :
la collecte d'un ensemble de données historiques d'échantillons de sang pour l'individu, et de données historiques d'activité pour l'individu (340) ;
l'analyse des données historiques d'échantillons de sang et des données historiques d'activité pour l'individu ; et
la détermination s'il faut valider ou invalider une ou plusieurs corrélations dans le modèle de prédiction de mobilisation des cellules sanguines dérivé cliniquement concernant un ou plusieurs changements des taux de mobilisation pour une ou plusieurs cellules sanguines et une ou plusieurs modifications des caractéristiques de surface des cellules sanguines avec un ou plusieurs facteurs sélectionnés parmi les données personnelles, les données génétiques et les données d'activité basées sur un ou plusieurs changements réels des taux de mobilisation pour une ou plusieurs cellules sanguines et un ou plusieurs changements réels des caractéristiques de surface des cellules sanguines observés dans les données historiques d'échantillon de sang (360).

13. Support de stockage non transitoire lisible par ordinateur selon la revendication 10, dans lequel les instructions amènent en outre le processeur à effectuer les étapes de :
inspection des données d'activité de pré-test pour l'individu ;
détection d'une incohérence dans les données d'activité de pré-test (720) ;
réception de données d'activité de pré-test supplémentaires à partir d'un capteur télémétrique supplémentaire (730) ; et
remplacement des données d'activité de pré-test en provenance du capteur télémétrique par les données d'activité de pré-test supplémentaires en provenance du capteur télémétrique supplémentaire (740).
